# FASCICULE DE BREVET EUROPEEN

(11) **EP 2 381 939 B1**
(45) Date de publication et mention de la délivrance du brevet: **08.04.2015**
(21) Numéro de dépôt: 10706291.1
(22) Date de dépôt: 11.01.2010
(51) Int. Cl.: A61K 8/365, A61K 31/191, A61P 17/02, A61Q 1/14, A61Q 19/08

(54) **COMPOSITIONS COSMETIQUES COMPRENANT DES DERIVES D'ACIDE CETOGLUCONIQUE**
KETOCLUCONSAÜRE-DERIVATE ENTHALTENDEN KOSMETIKZUSAMMENSETZUNGEN
COSMETIC COMPOSITIONS COMPRISING KETOGLUCONIC ACID DERIVATIVES

(30) Priorité: 29.01.2009 FR 0900383
(43) Date de publication de la demande: 02.11.2011
(73) Titulaire: Soliance, 51110 Pomacle (FR)
(72) Inventeur: REYNAUD, Romain, F-51100 Reims (FR); RANNOU, Alexis, F-02860 Vorges (FR); DINANT, Céline, F-51490 Saint-Hilaire-le-Petit (FR); LAFOSSE, Frédéric, F-51100 Reims (FR)
(74) Mandataire: Grosset-Fournier, Chantal Catherine
(86) Numéro de dépôt international: PCT/FR2010/050031
(87) Numéro de publication internationale: WO 2010/086534

(56) Documents cités:
- DEMINA N I ET AL: "INFLUENCE OF BALIS-2 AND SOME OF ITS COMPONENTS ON THE STRUCTURAL EPIDERMIS VARIATION OF FULLTHICKNESS WOUND HEALING OF RABBIT EAR SKIN" DOKLADY AKADEMII NAUK. ROSSIJSKA AKADEMI NAUK, NAUKA, MOSCOW, RU, vol. 363, no. 2, 1 novembre 1998 (1998-11-01), pages 274-277, XP009133971 ISSN: 0869-5652
- GUPTA A ET AL: "GLUCONOBACTER OXYDANS: ITS BIOTECHNOLOGICAL APPLICATIONS" JOURNAL OF MOLECULAR MICROBIOLOGY AND BIOTECHNOLOGY, HORIZON SCIENTIFIC PRESS, WYMONDHAM, GB, vol. 3, no. 3, 1 juillet 2001 (2001-07-01) , pages 445-456, XP008026573 ISSN: 1464-1801 cité dans la demande
- "Abstracts presented at the Third European Symposium on Elastin, held in Manchester, UK, June 30-July 3, 2004" MATRIX BIOLOGY, ELSEVIER LNKD- DOI:10.1016/J.MATBIO.2004.08.002, vol. 23, no. 6, 1 octobre 2004 (2004-10-01), pages 393-415, XP004632662 ISSN: 0945-053X

## Description

La présente invention concerne des compositions cosmétiques contenant au moins un dérivé d'acide cétogluconique et leur utilisation en tant qu'agent antivieillissement et restructurant de la peau.

Chez les mammifères en général, particulièrement chez l'Homme, la peau est constituée de deux parties principales, à savoir une couche externe, l'épiderme et une couche interne, le derme.

L'épiderme assure l'imperméabilité de la peau et sa résistance. Elle se renouvelle toutes les quatre semaines environ par l'élimination des cellules mortes superficielles. L'épiderme est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes dispersés dans un milieu complexe, appelé matrice extracellulaire, composée principalement de fibres de collagène, d'élastine, d'acide hyaluronique et des protéoglycanes**.**

L'élastine apporte élasticité et tonicité et l'acide hyaluronique donne du volume et participe à l'hydratation.

Au fil des jours, la peau vieillit ce qui se manifeste d'abord par l'apparition de ridules puis de rides notamment sur le visage et/ou par un affaissement de la peau. Le vieillissement affecte d'abord l'épiderme dont l'épaisseur se réduit. Le pouvoir de division cellulaire dans sa couche basale diminue et le temps de renouvellement de la couche cornée superficielle se rallonge. La maturation de ces cellules est imparfaite et la kératinisation n'aboutit plus à créer une couche cornée régulière et homogène. On note concomitamment une désorganisation de la partie profonde de la peau, un ralentissement du renouvellement cellulaire ainsi qu'une diminution de la production d'élastine.

Outre la cause chronobiologique du vieillissement, il existe également des causes dites externes telles que le soleil, le tabac, le manque de sommeil, les régimes alimentaires déficients en vitamines. Par ailleurs, on sait aussi que l'altération de la production hormonale (essentiellement les carences en oestrogènes) associée à la ménopause accentue le vieillissement cutané. Elle contribue à la diminution de l'élastine et de l'acide hyaluronique dans le derme et à l'amoindrissement du renouvellement des cellules de l'épiderme. La peau est ainsi moins souple, moins épaisse, avec une sensation de sécheresse et d'affaissement Les éléments structurants de la peau comme l'élastine et l'acide hyaluronique sont parmi les premiers responsables de notre apparence. En effet, les modifications du taux d'élastine et d'acide hyaluronique sont responsables de la perte d'élasticité, des rides et de l'affaissement de la peau. C'est en partie sur eux qu'agissent les produits cosmétiques.

Depuis de nombreuses années, on connaît des produits cosmétiques pour combattre le vieillissement de la peau mais aucun n'est satisfaisant à ce jour.

Dans la publication DEMINA N et al. : "Influence of Balis-2 and some of its components on the structural epidermis variation of fullthickness wound healing of rabbit ear skin » Doklady Akademii Nauk. Rossijska Akademi Nauk, Nauka, Moskau, RU, vol. 363, n°2,1 novembre 1998 (1998-11-01), pages 274-277, XP009133971 ISSN:0869-5652, les auteurs ont étudié les propriétés cicatrisantes d'une préparation de « Balis-2 et de certains de c=ses constituants pris isolément dont l'acide 5-céto-gluconique en solution.

Or de façon surprenante, les inventeurs ont découvert que des dérivés d'acide cétogluconique ont un effet stimulateur sur la néosynthèse de l'élastine et de l'acide hyaluronique donc pourront avoir un effet bénéfique sur la peau.

La présente invention a donc pour objet une composition pour application topique contenant dans un milieu cosmétiquement acceptable, au moins un composé de formule (1) 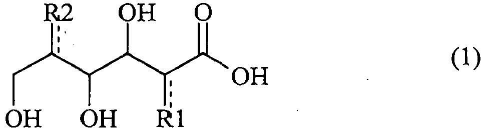 dans laquelle
R1 et R2 représentent indépendamment l'un de l'autre un atome d'oxygène ou groupe hydroxy et -̅ -̅ -̅ -̅ représente une double liaison lorsque R1 ou R2 représente un atome d'oxygène ou une liaison simple quand R1 ou R2 représente un groupe hydroxy, à condition que R1 et R2 ne représentent pas simultanément un groupe hydroxy,
ledit composé étant sous forme libre ou sous forme de sel cosmétiquement acceptable et présent en une quantité telle qu'il présente une activité stimulante de la néosynthèse d'élastine et une activité stimulante de la néosynthèse d'acide hyaluronique.

Les composés de formule (1) peuvent être sous forme de racémate, sous forme (L) ou sous forme (D).

Dans un mode avantageux de réalisation de l'invention, les composés de formule (1) sont des sels solubles dans l'eau qui sont choisis parmi les sels de potassium et de calcium.

Dans un autre mode de réalisation de l'invention, les composés de formule (1) sont le sel de potassium de l'acide 5-céto-(D)-gluconique de formule (1a) 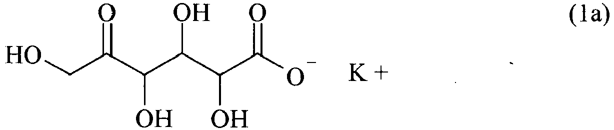 ou le sel de calcium de l'acide 2-céto-(D)-gluconique de formule (1b)

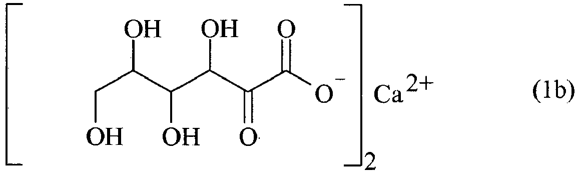

Les composés de formule (1) sont disponibles dans le commerce ou peuvent être préparés selon des techniques décrites dans la littérature ou connues de l'homme du métier à partir de composés disponibles dans le commerce. Ils peuvent être produits par exemple par *Gluconobacter oxydans* comme décrit par Gupta A. et al. (J. Mol. Microbiol. Biotechnol., (2001), 3(3), 445-456.

Conformément à l'invention les compositions peuvent se présenter sous toute forme adaptée à une utilisation topique, notamment sous forme d'une émulsion simple huile dans l'eau ou eau dans l'huile ou d'une émulsion multiple eau/huile/eau ou huile/eau/huile, d'un gel, d'une lotion ou sous forme d'une dispersion de sphérules. Ces compositions sont préparées selon des méthodes usuelles dans le domaine cosmétique.

Dans la composition selon l'invention, le au moins un composé de formule (1) représente avantageusement de 0,0001 % à 10 % en poids par rapport au poids total de la composition, avantageusement entre 0,001 % à 1 % ou 0,01 % à 0,5 % ou 0,1 % à 0,25 % et peut être associé à des adjuvants hydrophiles ou lipophiles habituellement utilisés.

A titre d'exemple d'adjuvants on peut citer notamment les tensioactifs, les gélifiants, les conservateurs, les parfums, les charges, les matières colorantes et les actifs autres que les composés de formule (1).

L'invention a également pour objet l'utilisation de la composition selon l'invention pour protéger la peau contre les symptômes liés à la diminution des concentrations en acide hyaluronique et /ou en élastine.

L'invention a également pour objet l'utilisation de la composition selon l'invention pour tonifier, hydrater, protéger, et/ou raffermir la peau.

L'invention concerne également un procédé de traitement cosmétique de la peau, caractérisé en ce qu'il consiste à appliquer sur la peau une composition selon l'invention et l'utilisation d'un composé de formule (1) comme principe actif stimulant la production d'acide hyaluronique et/ou d'élastine, dans une composition cosmétique.

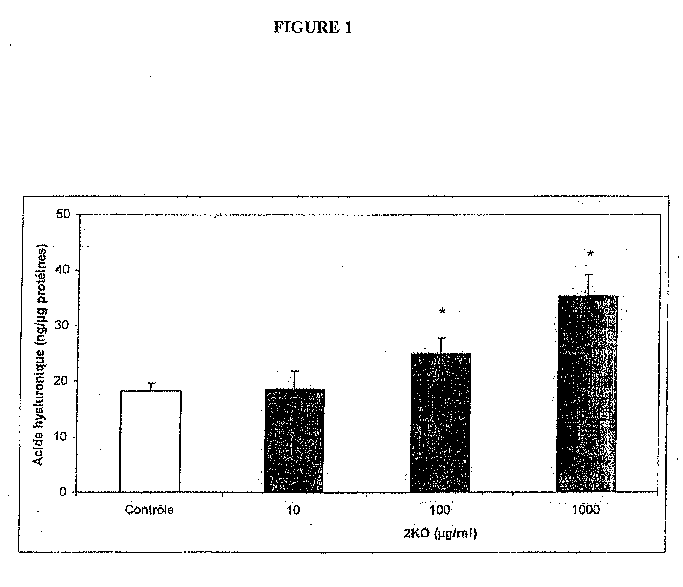

L'invention sera illustrée à l'aide des exemples 1 à 3 et des figures 1 à 4 qui suivent.
La figure 1 illustre l'effet du sel de calcium de l'acide 2-céto-(D)-gluconique (2KO) à différentes concentrations sur la production d'acide hyaluronique dans un modèle de fibroblastes dermiques humains normaux adultes après 24 heures d'incubation selon le protocole de l'exemple 1. * p<0,05 test de Student

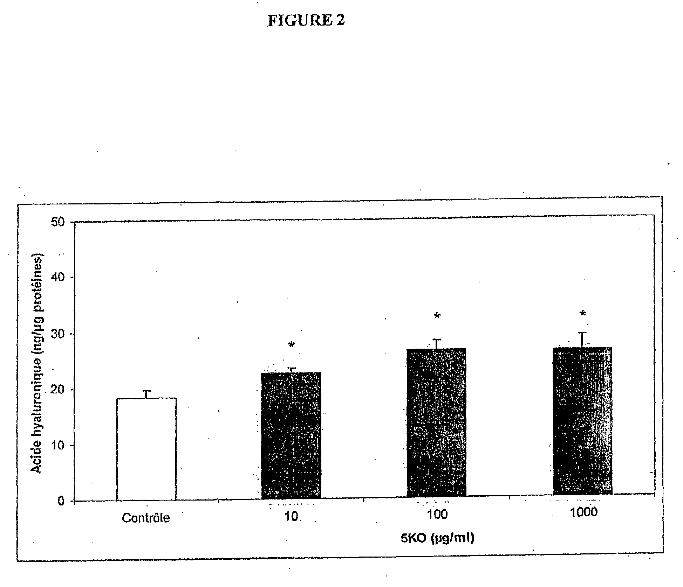

La figure 2 illustre l'effet du sel de potassium de l'acide 5-céto-(D)-gluconique (5KO) à différentes concentrations sur la production d'acide hyaluronique dans un modèle de fibroblastes dermiques humains normaux adultes après 24 heures d'incubation selon le protocole de l'exemple 1. * p<0,05 test de Student

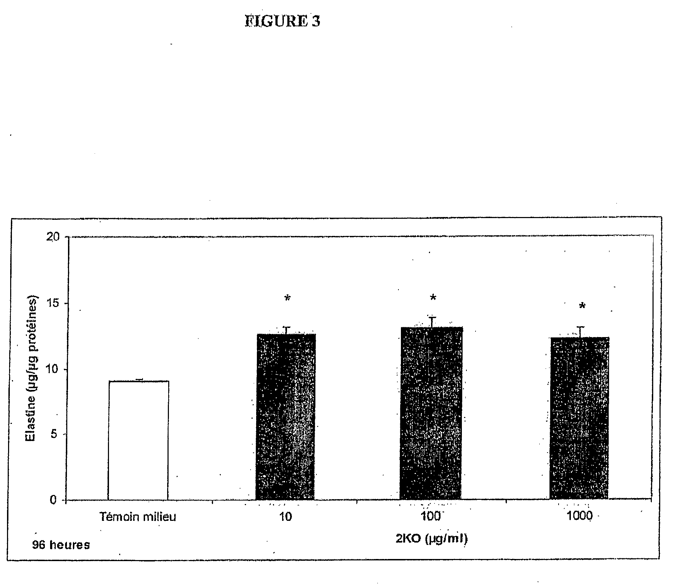

La figure 3 illustre l'effet du sel de calcium de l'acide 2-céto-(D)-gluconique (2KO) à différentes concentrations sur la production d'élastine dans un modèle de fibroblastes dermiques humains normaux adultes après 96 heures d'incubation selon le protocole de l'exemple 2. * p<0,05 test de Student.

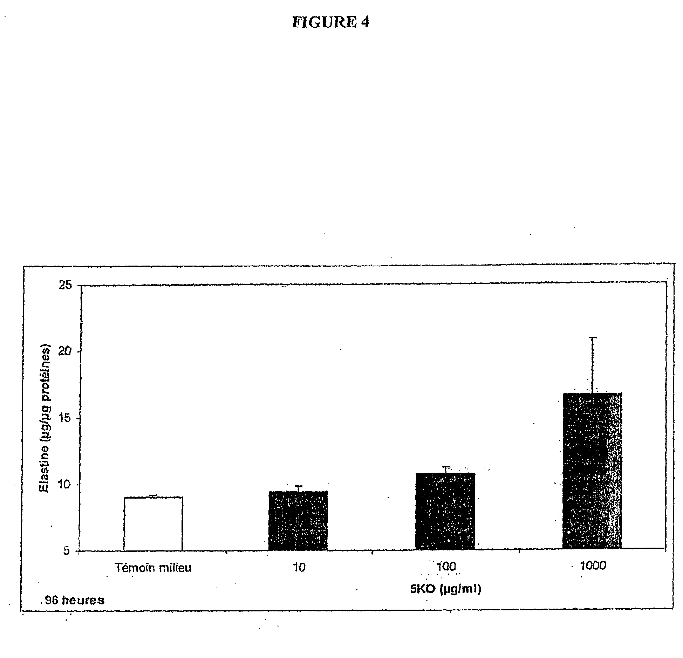

La figure 4 illustre l'effet du sel de potassium de l'acide 5-céto-(D)-gluconique (5KO) à différentes concentrations sur la production d'élastine dans un modèle de fibroblastes dermiques humains normaux adultes après 96 heures d'incubation selon le protocole de l'exemple 2. * p<0,05 test de Student

### EXEMPLE 1: EFFET DU SEL DE CALCUM DE L'ACIDE 2-CETO-(D)-GLUCONIQUE (2KO) ET DU SEL DE POTASSIUM DE L'ACIDE 5-CETO-(D)-GLUCONIQUE (5KO) SUR LA NEOSYNTHESE D'ACIDE HYALURONIQUE DANS UN MODELE DE FIBROBLASTES DERMIQUES HUMAINS NORMAUX ADULTES CULTIVES EN MONOCOUCHES

### I. MATERIEL ET METHODES

### I.1- Produits à tester

Le sel de calcium de l'acide 2-céto-(D)-gluconique et le sel de potassium de l'acide 5-céto-(D)-gluconique ont été préparés par les inventeurs selon la technique décrite par Gupta A*. et al.* (déjà cité) et adaptée au laboratoire et ont été conservés à température ambiante jusqu'à leur utilisation.

### I. 2 - Cultures

Des monocouches confluentes de fibroblastes dermiques humains normaux ont été obtenues en cultivant des cellules issues d'une plastie abdominale réalisée chez une femme de 33 ans. Les fibroblastes sont isolés en utilisant une digestion enzymatique à l'aide de dispase et de collagénase puis cultivés dans un milieu DMEM (Dulbecco's Modified Eagle Médium) et Ham's F12 dans un rapport (1:1) contenant 10 % se sérum de veau foetal, des antibiotiques et de l'amphotéricine B.

### I. 3 - Produit de référence

L'activateur de référence utilisé est le Facteur de croissance transformant (Transforming Growth Factor P ou TGF-β) à une concentration de 10 ng/ml.

### I. 4 - Incubation des produits

Les fibroblastes ont été incubés 24 heures à 37 °C, sous atmosphère humide et 5 % de CO₂, en absence (milieu de culture seul) ou en présence du produit de référence, ou de concentrations croissantes de produits à tester (10 ; 100 et 1000 µg/ml) pour les deux produits. Les produits à tester ont été solubilisés directement dans le milieu de culture.

### I. 5 - Evaluation des effets

### I 5.1 - Dosage d'acide hyaluronique

A la fin de la période d'incubation, l'acide hyaluronique contenu dans les milieux de culture a été quantifié à l'aide d'un kit E.L.I.S.A. sensible et spécifique.

### I. 5. 2 - Dosage des protéines

A la fin de la période d'incubation, les protéines contenues dans les lysats cellulaires ont été quantifiées par la méthode spectrocolorimétrique de Bradford.

### I. 6 - Statistiques

Les résultats sont donnés sous la forme de ng d'acide hyaluronique par µg de protéines totales du tapis cellulaire (moyennes +/- la déviation standard, S.D.). La significativité statistique des différences observées entre les conditions « Contrôle » et « Produit de référence », a été évaluée par un test de Student *(Student t-test).* La significativité statistique des différences observées entre les conditions «Contrôle» et « Produits à tester », a été évaluée produit par produit, par une analyse de la variance à un facteur (One Way ANOVA ou One Way Anova on Ranks+), suivie, lorsque cela était nécessaire, d'un test de Holm-Sidak ou de Dunn's (* : p<0,05).

### II. RESULTATS

### II. 1 - Produits à tester

### II. 1. 1 - Sel de calcium de l'acide 2-céto-(D)-gluconique

Les résultats sont donnés dans le tableau 1 ci-dessous et dans figure 1.

**Tableau 1 : effet du sel de calcium de l'acide 2-céto-(D)-gluconique (2KO) à différentes concentrations sur la production d'acide hyaluronique dans un modèle de fibroblastes dermiques humains normaux adultes après 24 heures d'incubation**

| | Contrôle | Sel de calcium de l'acide 2-céto-(D)-gluconique (2 KO) µg/ml | | |
|---|---|---|---|---|
| | | 10 | 100 | 1000 |
| Moyenne | 18,3 | 18,5 | 25,0* | 35,3* |
| Déviation standard | 1,3 | 3,4 | 2,8 | 3,7 |
| % contrôle | 100 | 101,2 | 136,3 | 192,7 |

| | | | | |
|---|---|---|---|---|
| * moyenne significativement différente de celle du groupe contrôle (p<0,05) test de Student | | | | |

Ces résultats montrent que dans les conditions expérimentales retenues le sel de calcium de l'acide 2-céto-(D)-gluconique induit une augmentation significative de la néosynthèse d'acide hyaluronique : + 36,3 % (p<0,05) pour une concentration de 100 µg/ml et + 92,7 % (p<0,05) pour une concentration de 1000 µg/ml.

### II. 1.2 - Sel de potassium de l'acide 5-céto-(D)-gluconique

Les résultats sont présentés dans le tableau 2 ci-dessous et la figure 2.

**Tableau 2 : effet du sel de potassium de l'acide 5-céto-(D)-gluconique (5KO) à différentes concentrations sur la production d'acide hyaluronique dans un modèle de fibroblastes dermiques humains normaux adultes après 24 heures d'incubation**

| | Contrôle | Sel de potassium de l'acide 5-céto-(D)-gluconique (5 KO) µg/ml | | |
|---|---|---|---|---|
| | | 10 | 100 | 1000 |
| Moyenne | 18,3 | 22,6* | 26,6* | 26,4* |
| Déviation standard | 1,3 | 0,8 | 1,8 | 2,7 |
| % contrôle | 100 | 123,3 | 144,9 | 144,0 |

| | | | | |
|---|---|---|---|---|
| * moyenne significativement différente de celle du groupe contrôle (p<0,05) test de Student | | | | |

Ces résultats montrent que dans les conditions expérimentales retenues le sel de potassium de l'acide 5-céto-(D)-gluconique induit une augmentation significative de la néosynthèse d'acide hyaluronique : + 23,3 % (p<0,05) pour une concentration de 10 µg/ml, + 44,9 % (p<0,05) pour une concentration de 100 µg/ml et + 44,0 % (p<0,05) pour une concentration de 1000 µg/ml.

### II .1.3 Produit de référence

Les résultats sont donnés dans le tableau 3 ci-dessous.

**Tableau 3 : effet du facteur de croissance transformant (TGF-β) à la concentration de 10 ng/ml sur la production d'acide hyaluronique dans un modèle de fibroblastes dermiques humains normaux adultes après 24 heures d'incubation**

| | Contrôle | TGF-β 10ng/ml |
|---|---|---|
| Moyenne | 18,3 | 41,5*** |
| Déviation standard | 1,3 | 2,1 |
| % contrôle | 100 | 226,3 |

| | | |
|---|---|---|
| *** moyenne significativement différente de celle du groupe contrôle (p<0,001) test ANOVA | | |

Dans ces conditions, le produit de référence augmente significativement la production fibroblastique d'acide hyaluronique de 126,3 % (p < 0,001) à la dose testée de 10 ng/ml.

### EXEMPLE 2: EFFET DU SEL DE CALCUM DE L'ACIDE 2-CETO-(D)-GLUCONIQUE (2KO) ET DU SEL DE POTASSIUM DE L'ACIDE 5-CETO-(D)-GLUCONIQUE (5KO) SUR LA NEOSYNTHESE D'ELASTINE DANS UN MODELE DE FIBROBLASTES DERMIQUES HUMAINS NORMAUX ADULTES CULTIVES EN MONOCOUCHES

### I. MATERIEL ET METHODES

### I. 1 - Produits à tester

Le sel de calcium de l'acide 2-céto-(D)-gluconique et le sel de potassium de l'acide 5-céto-(D)-gluconique ont été préparés par les inventeurs selon la technique décrite par Gupta A*. et al.* (déjà cité) et adaptée au laboratoire et ont été conservés à température ambiante jusqu'à leur utilisation.

### I. 2 - Cultures

Des monocouches confluentes de fibroblastes dermiques humains normaux ont été obtenues en cultivant des cellules issues d'une plastie abdominale réalisée chez une femme de 33 ans. Des monocouches confluentes de fibroblastes dermiques humains normaux ont été obtenues en cultivant des cellules issues d'une plastie abdominale réalisée chez une femme de 33 ans. Les fibroblastes sont isolés en utilisant une digestion enzymatique à l'aide de dispase et de collagénase puis cultivés dans un milieu DMEM (Dulbecco's Modified Eagle Medium) et Ham's F12 dans un rapport (1:1) contenant 10 % se sérum de veau foetal, des antibiotiques et de l'amphotéricine B.

### I. 3 - Produit de référence

L'activateur de référence utilisé est l'acide ascorbique à une concentration de 100 µg/ml.

### I. 4 - Incubation des produits

Les fibroblastes ont été incubés 96 heures à 37 °C, sous atmosphère humide et 5 % de CO2, en absence (milieu de culture seul) ou en présence du produit de référence, ou de concentrations croissantes de produits à tester (10 ; 100 et 1000 µg/ml) pour les deux produits Les produits à tester ont été solubilisés directement dans le milieu de culture.

### I. 5 - Evaluation des effets

### I 5. 1 - Dosage de l'élastine

A la fin de la période d'incubation, l'élastine contenue dans les milieux de culture a été quantifiée par une méthode spectrocolorimétrique.

### I. 5. 2 - Dosage des protéines

A la fin de la période d'incubation, les protéines contenues dans les lysats cellulaires ont été quantifiées par une méthode spectrocolorimétrique de Bradford.

### I. 6 - Statistiques

Les résultats sont donnés sous la forme de ng d'élastine par µg de protéines totales du tapis cellulaire (moyennes +/- la déviation standard, S.D.). La significativité statistique des différences observées entre les conditions « Contrôle » et « Produit de référence », a été évaluée par un test de Student (*Student t-test*). La significativité statistique des différences observées entre les conditions «Contrôle» et « Produits à tester », a été évaluée produit par produit, par une analyse de la variance à un facteur (One Way ANOVA ou One Way Anova on Ranks+), suivie, lorsque cela était nécessaire, d'un test de Holm-Sidak ou de Dunn's (* : p<0,05).

### II. RESULTATS

### II. 1 - Produits à tester

### II.1. 1 - Sel de calcium de l'acide 2-céto-(D)-gluconique

Les résultats sont donnés dans le tableau 4 ci-dessous et dans figure 3.

**Tableau 4 : effet du sel de calcium de l'acide 2-céto-(D)-gluconique (2KO) à différentes concentrations sur la production d'élastine dans un modèle de fibroblastes dermiques humains normaux adultes après 96 heures d'incubation**

| | Contrôle | Sel de calcium de l'acide 2-céto-(D)-gluconique (2 KO) µg/ml | | |
|---|---|---|---|---|
| | | 10 | 100 | 1000 |
| Moyenne | 9,0 | 12,6* | 13,1* | 12,3* |
| Déviation standard | 0,1 | 0,5 | 0,8 | 0,8 |
| % contrôle | 100 | 139,7 | 144,6 | 135,8 |

| | | | | |
|---|---|---|---|---|
| * moyenne significativement différente de celle du groupe contrôle (p<0,05) test de Student | | | | |

Ces résultats montrent que dans les conditions expérimentales retenues le sel de calcium de l'acide 2-céto-(D)-gluconique induit une augmentation significative de la néosynthèse d'élastine + 39,7 % (p<0,05) pour une concentration de 10 µg/ml, + 44,6 % (p<0,05) pour une concentration de 100 µg/ml et + 35,8 % pour une concentration de 1000 µg/ml (p<0,05)

### II.1.2 - Sel de potassium de l'acide 5-céto-(D)-gluconique

Les résultats sont présentés dans le tableau 5 ci-dessous et la figure 4.

**Tableau 5 : effet du sel de potassium de l'acide 5-céto-(D)-gluconique (5KO) à différentes concentrations sur la production d'élastine dans un modèle de fibroblastes dermiques humains normaux adultes après 96 heures d'incubation**

| | Contrôle | Sel de potassium de l'acide 5-céto-(D)-gluconique (5KO) µg/ml | | |
|---|---|---|---|---|
| | | 10 | 100 | 1000 |
| Moyenne | 9,0 | 9,5 | 10,7 | 16,7* |
| Déviation standard | 0,1 | 0,4 | 0,5 | 4,2 |
| % contrôle | 100 | 104,8 | 118,9 | 184,4 |

| | | | | |
|---|---|---|---|---|
| * moyenne significativement différente de celle du groupe contrôle (p<0,05) test de Student | | | | |

Ces résultats montrent que dans les conditions expérimentales retenues le sel de potassium de l'acide 5-céto-(D)-gluconique induit une augmentation de la néosynthèse d'élastine : + 18,9 % (non significatif) pour une concentration de 100 µg/ml, + 84,4 % (p<0,05) pour une concentration de 1000 µg/ml.

### II .1.3 Produit de référence

Les résultats sont donnés dans le tableau 6 ci-dessous

**Tableau 6 : effet de l'acide ascorbique à la concentration de 100 µg/ml sur la production d'élastine dans un modèle de fibroblastes dermiques humains normaux adultes après 96 heures d'incubation**

| | Contrôle | Acide ascorbique 100µg/ml |
|---|---|---|
| Moyenne | 9,0 | 12,7*** |
| Déviation standard | 0,1 | 0,9 |
| % contrôle | 100 | 140,3 |

| | | |
|---|---|---|
| *** moyenne significativement différente de celle du groupe contrôle (p<0,01) test ANOVA | | |

Dans ces conditions, le produit de référence (acide ascorbique) augmente significativement la production fibroblastique d'élastine de 40,3 % (p < 0,01).à la dose testée de 100 µg/ml.

Les résultats montrent que le sel de calcium de l'acide 2-céto-(D)-gluconique et le sel de potassium de l'acide 5-céto-(D)-gluconique peuvent être utilisés comme agents cosmétiques destinés à stimuler la synthèse d'élastine et d'acide hyaluronique par les fibroblastes du derme, en vue d'améliorer en particulier l'élasticité cutanée, la tonicité cutanée ou de favoriser le raffermissement cutané dans des produits anti-âge.

### EXEMPLE 3 : Exemples de formulations

Des formules cosmétiques contenant du sel de potassium de l'acide 5-céto-(D)-gluconique ou du sel de calcium de l'acide 2-céto-(D)-gluconique peuvent être préparées comme indiqué ci-dessous. Dans ces formules, les compositions sont exprimées en pourcentage en poids.

### 3.1 Emulsion anti-âge

| Ingrédients | % P/P |
|---|---|
| Xyliance® | 3% |
| Stearic acid | 0,5% |
| Dimethicone | 3% |
| Kendi oil® | 7% |
| Conservateur | qS |
| Dry Flo PC® | 2% |
| Eau | QSP 100% |
| Glycérine | 5% |
| Cosmedia ATH® | 2% |
| Sel de potassium de l'acide 5-céto-(D)-gluconique | 0,1% |
| Parfum | 0,2% |

### 3.2. Gel tenseur anti-âge

| Ingrédients | % P/P |
|---|---|
| Pemulen TR2® | 1% |
| Eau | Qsp 100% |
| Conservateur | qs |
| Glycérine | 2% |
| Sodium hydroxide | qs pH =7 |
| Easyliance® | 3% |
| Sel de calcium de l'acide 2-céto-(D)-gluconique | 0,1% |
| Parfum | qs |

### 3.3 Lotion Démaquillante tonique

| Ingrédients | % P/P |
|---|---|
| Eau | QSP 100% |
| Betaphroline® | 0,5% |
| Natrosol 250 HHR® | 0,1% |
| Vitalhyal® | 1% |
| Sel de potassium de l'acide 5-céto-(D)-gluconique | 0,1% |
| Eau de bleuet | 1% |
| Tween 20® | 2,5% |
| Conservateur | qs |
| Parfum | qs |
| Glycerine | 2% |
| Juazirine® | 1% |
| Colorants | qs |

## Revendications

1. Utilisation d'un composé de formule (1) 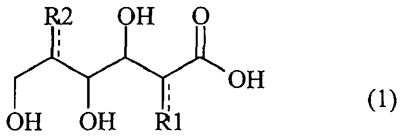 dans laquelle
R1 et R2 représentent indépendamment l'un de l'autre un atome d'oxygène ou groupe hydroxy et -̅ -̅ -̅ -̅ représente une double liaison lorsque R1 ou R2 représente un atome d'oxygène ou une liaison simple quand R1 ou R2 représente un groupe hydroxy, à condition que R1 et R2 ne représentent pas simultanément un groupe hydroxy,
ledit composé étant sous forme libre ou sous forme de sel cosmétiquement acceptable comme principe actif stimulant la production d'acide hyaluronique et/ou d'élastine, dans une composition cosmétique.

2. Utilisation d'un composé de formule (1) telle que définie à la revendication 1 comme principe actif pour tonifier, hydrater, protéger, et/ou raffermir la peau dans une composition cosmétique.

3. Utilisation selon l'une des revendications 1 ou 2 **caractérisée en ce que** au moins un composé de formule (1) est sous la forme de racémate, sous la forme (D) ou sous la forme (L).

4. Utilisation selon l'une quelconque des revendications précédentes **caractérisée en ce que** les sels sont choisis parmi les sels de potassium et de calcium.

5. Utilisation selon l'une quelconque des revendications précédentes **caractérisée en ce que** le composé de formule (1) est choisi dans le groupe comprenant le sel de potassium de l'acide 5-céto-(D)-gluconique (la) 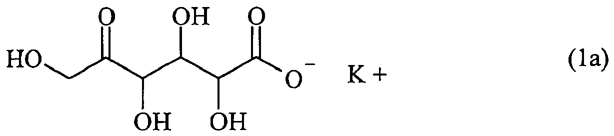 et le sel de calcium de l'acide 2-céto-(D)-gluconique de formule (1b)

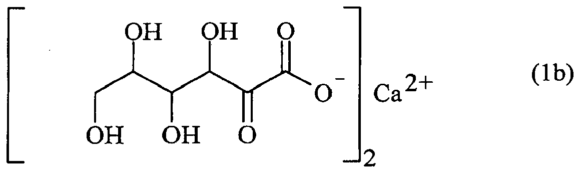

6. Utilisation selon l'une quelconque des revendications précédentes, **caractérisée en ce que** la composition se présente sous forme d'une émulsion, d'un gel, d'une lotion ou sous forme d'une dispersion de sphérules.

7. Utilisation selon l'une quelconque des revendications précédentes, **caractérisée en ce que** le au moins un composé de formule (1) représente de 0,0001 % à 10 % en poids par rapport au poids total de la composition.

8. Utilisation selon l'une quelconque des revendications précédentes, **caractérisée en ce qu'**elle contient aussi des adjuvants hydrophiles ou lipophiles.

9. Utilisation selon la revendication 8, **caractérisée en ce que** les adjuvants sont choisis parmi les gélifiants, les conservateurs, les parfums, les charges, les matières colorantes, les actifs autres que les composés de formule (1).

10. Composition pour application topique contenant dans un milieu cosmétiquement acceptable au moins un composé, ledit composé étant le sel de calcium de l'acide 2-céto-(D)-gluconique de formule (1b)

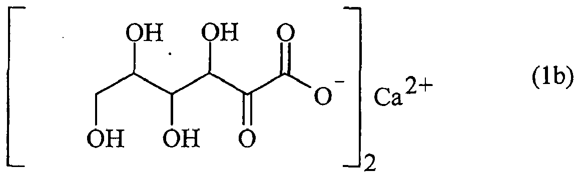

11. Utilisation cosmétique de la composition selon la revendication 10 pour tonifier, hydrater, protéger, et/ou raffermir la peau.

12. Procédé de traitement cosmétique de la peau, **caractérisé en ce qu'**il consiste à appliquer sur la peau une composition selon la revendication 10.

13. Procédé de traitement cosmétique pour tonifier, hydrater, protéger, et/ou raffermir la peau **caractérisé en ce qu'**il consiste à appliquer sur la peau une composition selon la revendication 10.

## Patentansprüche

1. Verwendung einer Verbindung der Formel (1) 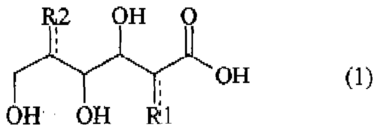 wobei R1 und R2 unabhängig voneinander für ein Sauerstoffatom oder eine Hydroxygruppe stehen und -̅ -̅ -̅ -̅ für eine Doppelbindung steht, wenn R1 oder R2 für ein Sauerstoffatom stehen, oder eine Einfachbindung, wenn R1 oder R2 für eine Hydroxygruppe stehen, unter der Bedingung, dass R1 und R2 nicht gleichzeitig für eine Hydroxygruppe stehen,
wobei die Verbindung in freier Form oder in Form eines kosmetisch akzeptablen Salzes vorliegt, als Wirkstoff, der die Produktion von Hyaluronsäure und/oder Elastin stimuliert, in einer Kosmetikzunammensetzung,

2. Verwendung einer Verbindung der Formel (1), wie in Anspruch 1 definiert, als Wirkstoff zum Beleben, Hydratisieren, Schützen und/oder Festigen der Haut in einer Kosmetiksusammensetzung.

3. Verwendung nach einem der Ansprüche 1 oder 2, **dadurch gekennzeichnet, dass** mindestens eine Verbindung der Formel (1) in Form eines Racemats in (D)- oder in (L)-Form vorliegt.

4. Verwendung nach einem der vorhergehenden Ansprüche, **dadurch gekennzeichnet, dass** die Salze aus den Kalium- und Calciumsalzen ausgewählt sind.

5. Verwendung nach einem der vorhergehenden Ansprüche, **dadurch gekennzeichnet, dass** die Verbindung der Formel (1) ausgewählt ist aus der Gruppe, umfassend das Kaliumsalz der 5-Keto-(D)-gluconsäure (la) 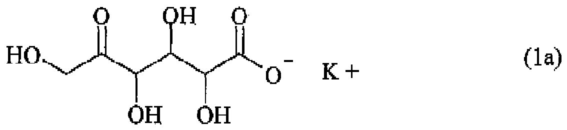 und das Calciumsalz der 2-Keto-(D)-gluconsäure der Formel (1b)

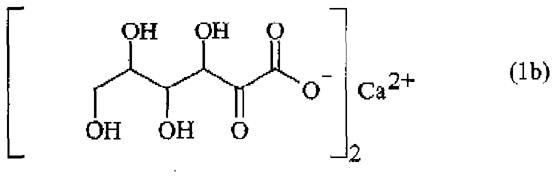

6. Verwendung nach einem der vorhergehenden Ansprüche, **dadurch gekennzeichnet, dass** die Zusammensetzung die Form einer Emulsion, eines Gels, einer Lotion oder die Form einer Kügelchendispersion aufweist.

7. Verwendung nach einem der vorhergehenden Ansprüche, **dadurch gekennzeichnet, dass** die mindestens eine Verbindung der Formel (1) 0,0001 Gew.-% bis 10 Gew.-%, bezogen auf das Gesamtgewicht der Zusammensetzung, darstellt.

8. Verwendung nach einem der vorhergehenden Ansprüche, **dadurch gekennzeichnet, dass** sie auch hydrophile oder lipophile Adjuvanzien enthält.

9. Verwendung nach Anspruch 8, **dadurch gekennzeichnet, dass** die Adjuvanzien ausgewählt sind aus Gelbildnern, Konservierungsmitteln, Parfums, Füllstoffen, Farbstoffen, anderen Wirkstoffen als die Verbindungen der Formel (1).

10. Zusammensetzung zum topischen Auftragen, enthaltend in einem kosmetisch akzeptablen Medium mindestens eine Verbindung, wobei es sich bei der Verbindung um ein Calciumsalz der 2-Keto-(D)-gluconsäure der Formel (1b) handelt:

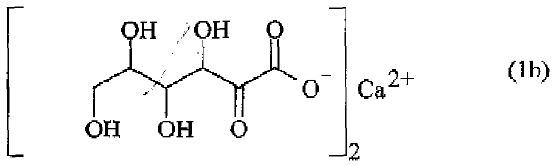

11. Kosmetische Verwendung der Zusammensetzung nach Anspruch 10 zum Beleben, Hydratisieren, Schützen und/oder Festigen der Haut.

12. Verfahren zur kosmetischen Behandlung der Haut, **dadurch gekennzeichnet, dass** es darin besteht, eine Zusammensetzung nach Anspruch 10 auf die Haut aufzutragen.

13. Verfahren zur kosmetischen Behandlung zum Beleben, Hydratisieren, Schützen und/oder Festigen der Haut, **dadurch gekennzeichnet, dass** es darin besteht, eine Zusammensetzung nach Anspruch 10 auf die Haut aufzutragen.

## Claims

1. Use of a compound of formula (1) 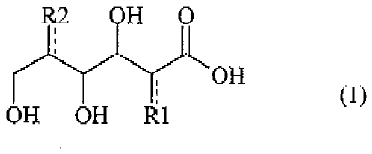 wherein
R1 and R2 represent each independently of the other an oxygen atom or a hydroxy group and -̅ -̅ -̅ -̅ represents a double bond when R1 or R2 represents a oxygen atom or a simple bond when R1 or R2 represents a hydroxy group, with the proviso that R1 and R2 do not represent simultaneously a hydroxy group,
said compound being under free form or under the form of a cosmetically acceptable salt, as an active ingredient stimulating the production of hyaluronic acid and/or of elastin in a cosmetic composition.

2. Use of a compound of formula (1) according to claim 1 as an active ingredient for tonifying, hydrating, protecting and/or hardening the skin in a cosmetic composition.

3. Use of a compound of formula (1) according to claim 1 or 2, **characterized in that** at least one compound of formula (1) is under the form of a racemate, under the (D) form or under the (L) form.

4. Use according to any of the preceding claims **characterized in that** the salts are selected from the potassium and calcium salts.

5. Use according to any of the preceding claims **characterized in that** the compound of formula (1) is selected from the group comprising the potassium salt of the 5-ceto-(D)-gluconic acid (1a) 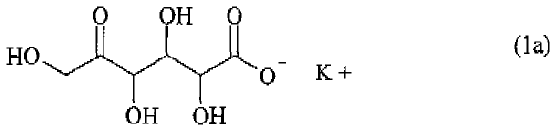 and the calcium salt of the 2-ceto-(D)-gluconic acid of formula (1b)

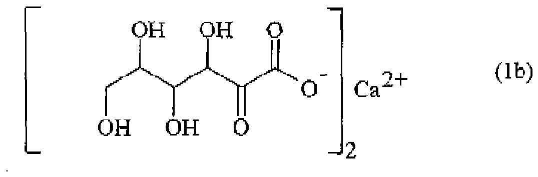

6. Use according to any of the preceding claims **characterized in that** the composition is under the form of an emulsion, a gel, a lotion or under the form of a dispersion of spheroids.

7. Use according to any of the preceding claims **characterized in that** the at least one compound of formula (1) represents from 0,0001 % to 10 % by weight relative to the total weight of the composition.

8. Use according to any of the preceding claims **characterized in that** it also contains hydrophilic or lipophilic additives.

9. Use according to claim 8, **characterized in that** the additives are selected from gelling agents, preservatives, perfumes, charges, pigments, active ingredients other than the compounds of formula (1).

10. Composition for topical application containing in a cosmetically acceptable medium, at least one compound, said compound being a calcium salt of 2-ceto-(D)-gluconic acid of formula (1b)

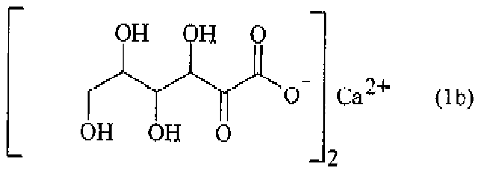

11. Cosmetic use of the composition according to claim 10 for tonifying, hydrating, protecting and/or hardening the skin.

12. Cosmetic treatment process of the skin **characterized in that** said proceed consisting in applying on the skin a composition according to claim 10.

13. Cosmetic treatment process for tonifying, hydrating, protecting and/or hardening the skin **characterized in that** it consists in applying on the skin a composition according to claim 10.
